# EUROPEAN PATENT APPLICATION

(11) **EP 4 269 429 A1**
(43) Date of publication of application: **01.11.2023**
(21) Application number: 21911559.9
(22) Date of filing: 22.12.2021
(51) Int. Cl.: C07K 14/495, C07K 7/08, C07K 7/06, A61K 38/00, A61P 35/00

(54) **NOVEL PEPTIDE CAPABLE OF INHIBITING TGF-BETA SIGNALING AND USE THEREOF**

(30) Priority: 23.12.2020 KR 20200182441
(71) Applicant: Korea Institute of Science and Technology, Seoul 02792 (KR)
(72) Inventor: JANG, Mi-Hue, Seoul 02792 (KR); YUK, Chae-Min, Seoul 02792 (KR); GO, Ga-Yeon, Seoul 02792 (KR); LEE, Young-Eun, Seoul 02792 (KR); SHIN, Sang-Chul, Seoul 02792 (KR)
(74) Representative: Schnappauf, Georg
(86) International application number: PCT/KR2021/019667
(87) International publication number: WO 2022/139493

(57) **Abstract**

The present invention relates to a novel peptide capable of inhibiting TGF-β signaling and the use thereof. The peptide of the present invention is capable of inhibiting the TGF-β-induced signaling pathway or the expression of TGF-β, and thus can treat or prevent various diseases caused by TGF-β signaling, and furthermore, can effectively inhibit tumorigenesis processes such as TGF-β-signaling induced tumor growth and metastasis.

## Description

### Technical Field

The present invention relates to a novel peptide capable of inhibiting TGF-β signaling and the use thereof.

### Background Art

TGF-β cytokines were discovered by their capacity to stimulate cell colony formation (Roberts A B, et al., Proc Natl Acad Sci USA 78: 5339-43, 1981); because this process is a classic marker of cellular transformation, the cytokine was called transforming growth factor beta (TGF-β), and TGF-β ligands (TGF-β1, TGF-β2, TGF-β3) are recognized as the prototype of multifunctional growth factors. TGF-β is known as an inhibitor of proliferation of epithelial, endothelial and hematopoietic cells, and as one of the most potent regulators of extracellular matrix production and deposition and of tissue repair cascade.

Meanwhile, TGF-β is known to be involved in tumor growth, invasion and metastasis in advanced cancer stages. Specifically, TGF-β is known to exhibit various activities in the tumorigenesis process by activating numerous SMAD-independent signaling pathways, including Ras-MAPK and PI3K-AKT, which regulate epithelial-mesenchymal transition (EMT) in tumor cells or other cells.

Therefore, TGF-β has recently been considered as a target for preventing tumor growth and metastasis, and various TGF-β inhibitors, including monoclonal antibodies against TGF-β, small molecule compounds (kinase inhibitors), antisense oligonucleotides (ASOs), and chimeric proteins, are currently being tested clinically. However, the development of therapeutic methods based on TGF-β signaling inhibition has progressed slowly for a long time.

In order to solve this problem, it is necessary to develop a therapeutic agent capable of effectively inhibiting the TGF-β signaling pathway.

### DISCLOSURE

### Technical Problem

One aspect provides a peptide derived from transforming growth factor-β (TGF-β) or an analog thereof.

Another aspect provides a polynucleotide encoding the peptide of the present invention.

Still another aspect provides an expression vector comprising the polynucleotide.

Yet another aspect provides a pharmaceutical composition for treating or preventing TGF-β-related disease, the pharmaceutical composition containing, as an active ingredient, the peptide, the polynucleotide or the expression vector.

Still yet another aspect provides a method for treating or preventing TGF-β-related disease, the method comprising a step of administering to a subject the pharmaceutical composition containing, as an active ingredient, the peptide, the polynucleotide or the expression vector.

A further aspect provides a pharmaceutical composition for treating or preventing cancer, the pharmaceutical composition containing, as an active ingredient, the peptide, the polynucleotide or the expression vector.

Another further aspect provides a method for treating or preventing cancer, the method comprising a step of administering to a subject the pharmaceutical composition containing, as an active ingredient, the peptide, the polynucleotide or the expression vector.

### Technical Solution

One aspect provides a peptide derived from transforming growth factor-β (TGF-β) or an analog thereof.

In one embodiment of the present invention, the peptide is capable of inhibiting TGF-β signaling.

As used herein, the term "transforming growth factor-β (TGF-β)" refers to a cytokine belonging to the TGF-β superfamily, and three TGF-β isoforms (TGF-β1, TGF-β2 and TGF-β3) are known to be expressed in mammals. Signal transduction by TGF-β plays a critical role in various biological processes and performs various functions such as cell growth arrest, apoptosis, differentiation, and epithelial-mesenchymal transition (EMT). The TGF-β signaling pathway is tightly regulated and plays a critical role in development and organogenesis as well as maintenance of cell homeostasis. Thus, disruption of TGF-β signaling can lead to life-threatening diseases such as cancer, fibrosis, and congenital malformations.

TGF-β is known to exhibit cancer inhibitory activity in the early stages of tumorigenesis, but promote cancer growth in the late stages of tumorigenesis. In particular, it is known that TGF-β1 is expressed in large amounts in most cancer tissues, and that cancer patients with high expression of TGF-β1 often have malignant cancer and also have poor prognosis. TGF-β secreted from cells initiates signal transduction by binding to a heteromeric complex of two types of receptors (type I and type II receptors). When TGF-β binds to the type II receptor, the type I receptor recognizes it and binds to the type II receptor. At this time, when the type II receptor phosphorylates the GS domain of the type I receptor, the type I receptor kinase is activated. The phosphorylated TGF-β type I receptor triggers activation of Smad2 and Smad3 by phosphorylating the C-terminal serine residues of TGF-β signaling mediators (Smad2 and Smad3). Activated Smad2 and Smad3 form complexes with Smad4 and translocate into the nucleus, participating in the expression of target genes.

In addition, it is known that TGF-β is a crucial enforcer of immune homeostasis and tolerance, regulating immune tolerance and inflammatory responses by inhibiting the expansion and function of many components of the immune system. In particular, it is known that TGF-β plays an important role as a mediator of immune suppression in the tumor microenvironment. Therefore, TGF-β is known to play an important role in promoting cancer growth in the tumor immune environment, and thus combination therapies of immunotherapeutic anticancer agents and various TGF-β signaling inhibitors have been studied and developed.

The peptide is capable of inhibiting TGF-β signaling by binding to TGF-β receptors (TGFBR1 and/or TGFBR2). Specifically, the peptide is capable of inhibiting TGF-β signaling by a mechanism in which it competes with TGF-β for binding to the TGF-β receptor, thereby preventing the TGF-β cytokine from binding to the TGF-β receptor.

In addition, the peptide is capable of inhibiting TGF-β signaling by inhibiting the expression level of TGF-β itself in cells. Specifically, the peptide is capable of inhibiting TGF-β signaling by a mechanism that reduces the expression level of TGF-β in cells through an auto-inhibition pathway or reduces the extracellular release of TGF-β.

In one embodiment of the present invention, the peptide may comprise an amino acid sequence represented by the following Formula 1, which is set forth in SEQ ID NO: 16.

[Formula 1] F-X₁-L-G-P-X₂-P-Y-I-W-X₃-L-D-T

wherein X₁, X₂ and X₃ may be each independently cysteine (C) or serine (S), and specifically, X₁-X₂-X₃ may be C-C-S, C-C-C, or S-S-S.

The peptide may additionally comprise any amino acid or peptide. Specifically, the peptide may comprise a peptide in which any additional amino acid or peptide is linked directly to one terminus, preferably the C-terminus, of the amino acid sequence represented by Formula 1. The additional amino acid or peptide may be alanine (A), phenylalanine (F), valine (V), leucine (L), serine (S), or a combination thereof, and non-limiting examples thereof include alanine (A), phenylalanine (F), valine-leucine-serine-leucine (V-L-S-L), or valine-leucine-serine-phenylalanine (V-L-S-F).

The peptide may comprise the amino acid sequence of at least one of SEQ ID NOs: 1 to 8, and specifically, may consist of the amino acid sequence of one of SEQ ID NOs: 1 to 8.

In one embodiment of the present invention, the peptide may comprise an amino acid sequence represented by the following Formula 2, which is represented by SEQ ID NO: 17.

[Formula 2] A-H-C-S-C-D

The peptide may additionally comprise any amino acid or peptide. Specifically, the peptide may be one in which any additional amino acid or peptide is linked directly to one terminus, preferably the C-terminus, of the amino acid sequence represented by Formula 2. The additional amino acid or peptide may be serine (S), arginine (R), aspartic acid (D), asparagine (N), glycine (G), alanine (A), phenylalanine (F), or a combination thereof, and non-limiting examples thereof include serine-arginine-aspartic acid-asparagine (S-R-D-N), or glycine-alanine-phenylalanine-alanine (G-A-F-A).

The peptide may comprise the amino acid sequence of at least one of SEQ ID NOs: 9 and 10, and specifically, may consist of the amino acid sequence of one of SEQ ID NOs: 9 and 10.

In one embodiment of the present invention, the peptide may comprise an amino acid sequence represented by the following Formula 3, which is represented by SEQ ID NO: 18.

[Formula 3] T-I-V-Y-Y-V-X₄-X₅-K-P-K-V-E-Q

wherein X₄ may be glycine (G), histidine (H) or valine (V), X₅ may be arginine (R), leucine (L), or isoleucine (I), and specifically, X₄-X₅ may be G-R, G-L, H-I, or V-I.

The peptide may comprise the amino acid sequence of at least one of SEQ ID NOs: 11 to 14, and specifically, may consist of the amino acid sequence of one of SEQ ID NOs: 11 to 14.

In the present invention, the amino acid sequences of the peptides include, without limitation, not only the amino acid sequence set forth in each of said SEQ ID NOs., but also any amino acid sequence which has a homology of at least 60%, at least 70%, at least 80%, at least 85%, at least 90%, at least 91%, at least 92%, at least 93%, at least 94%, at least 95%, at least 96%, at least 97%, at least 98% or at least 99% to the amino acid sequence set forth in each of said SEQ ID NOs., as long as the amino acid sequence is the amino acid sequence of a peptide exhibiting an efficacy that is substantially the same as or similar to the peptide set forth in each of said SEQ ID NOs. In addition, it is obvious that an amino acid sequence having a deletion, modification, substitution or addition of one or more amino acids also falls within the scope of the present invention, as long as it has homology to the above-described sequence and has a biological activity that is substantially the same as or similar to that of the peptide set forth in each of said SEQ ID NOs.

As used herein, the term "homology" refers to the degree of similarity between two nucleotide sequences encoding proteins or two amino acid sequences. When the homology is sufficiently high, the corresponding proteins or the expression products of the corresponding genes may have the same activity or similar activities. The homology may be expressed as a percentage depending on the degree of matching with a given amino acid sequence or nucleotide sequence. In the present specification, a homology sequence having an activity which is identical or similar to that of the given amino acid sequence or nucleotide sequence is presented as "% homology". For example, the homology sequence may be determined by, for example, a standard software, specifically, BLAST 2.0, which calculates parameters such as score, identity, similarity, etc., or by comparing the sequences in a hybridization experiment under defined stringent conditions, and appropriate hybridization conditions to be defined are within the skill of the art and may be determined by a method well known to those skilled in the art (e.g., J. Sambrook et al., Molecular Cloning, A Laboratory Manual, 2nd Edition, Cold Spring Harbor Laboratory press, Cold Spring Harbor,New York, 1989; F.M. Ausubel et al., Current Protocols in Molecular Biology, John Wiley & Sons, Inc., New York; Needleman, S.B. and Wunsch, CD., (1970), Journal of Molecular Biology, 48, 443-453).

According to one embodiment of the present invention, in the present invention, candidate peptides capable of inhibiting TGF-β signaling were constructed. These peptides are capable of inhibiting TGF-β signaling by binding to TGF-β receptors or by reducing the expression level or extracellular release of TGF-β. Based on the inhibition of TGF-β signaling, they can treat or prevent TGF-β signaling-related disease, and furthermore, can also effectively treat or prevent TGF-β expressing cancer.

Another aspect provides a polynucleotide encoding the peptide. Here, contents regarding "TGF-β" and "peptide derived from TGF-β" are as described above.

As used herein, the term "polynucleotide" refers to DNA encoding genetic information, which is a polymer substance consisting of nucleotides linked together.

In the present invention, the nucleotide sequences encoding the peptides include, without limitation, not only the nucleotide sequence encoding the amino acid sequence set forth in each of said SEQ ID NOs., but also any nucleotide sequence which has a homology of at least 60%, at least 70%, at least 80%, at least 85%, at least 90%, at least 91%, at least 92%, at least 93%, at least 94%, at least 95%, at least 96%, at least 97%, at least 98% or at least 99% to the nucleotide sequence encoding the amino acid sequence set forth in each of said SEQ ID NOs., as long as the nucleotide sequence is the nucleotide sequence encoding a peptide exhibiting an efficacy that is substantially the same as or similar to the peptide set forth in each of said SEQ ID NOs. In addition, it is obvious that a nucleotide sequence encoding an amino acid sequence having a deletion, modification, substitution or addition of one or more amino acids also falls within the scope of the present invention, as long as the sequence has homology to the above-described sequence and has a biological activity that is substantially the same as or similar to that of the peptide set forth in each of said SEQ ID NOs.

In addition, in the present invention, the polynucleotide encoding each of the peptides may undergo various modifications in the coding region within the scope that does not change the amino acid sequence of the protein expressed from the coding region, due to codon degeneracy or in consideration of the codons preferred in an organism in which the peptide is to be expressed. Thus, the polynucleotide may comprise, without limitation, any polynucleotide sequence encoding each of the peptides. In addition, the polynucleotide may comprise, without limitation, a probe that may be prepared from a known gene sequence, for example, any nucleotide sequence which may hybridize with a sequence complementary to all or part of the polynucleotide sequence under stringent conditions and encode a protein having the activity of the peptide.

The term "stringent conditions" refer to conditions which allow the specific hybridization between polynucleotides. Such conditions are disclosed in detail in the literature (e.g., J. Sambrook et al., supra). For example, the stringent conditions may include conditions under which genes having high homology, in particular, genes having at least 40%, specifically at least 90%, more specifically at least 95%, still more specifically at least 97%, particularly specifically at least 99% homology hybridize with each other, while genes having homology lower than the above homology do not hybridize with each other; or may include ordinary washing conditions of Southern hybridization, i.e., washing once, specifically twice or three times, at a salt concentration and temperature corresponding to 60°C, 1×SSC, and 0.1% SDS, specifically 60°C, 0.1×SSC, and 0.1% SDS, more specifically 68°C, 0.1×SSC, and 0.1% SSD.

Hybridization requires that two polynucleotides have complementary sequences, although mismatches between bases are possible depending on the stringency of the hybridization. The term "complementary" is used to describe the relationship between nucleotide bases that may hybridize with each other. For example, with respect to DNA, adenosine is complementary to thymine, and cytosine is complementary to guanine. Thus, the present invention may also include an isolated polynucleotide fragment complementary to the entire sequence as well as a polynucleotide sequence substantially similar thereto.

Specifically, a polynucleotide having homology may be detected using hybridization conditions including a hybridization step at a Tₘ value of 55°C under the above-described conditions. In addition, the Tₘ value may be 60°C, 63°C, or 65°C, without being limited thereto, and may be appropriately controlled by those skilled in the art depending on the purpose thereof.

The appropriate stringency for hybridizing polynucleotides depends on the length and degree of complementarity of the polynucleotides, and these variables are well known in the art (see Sambrook et al., supra, 9.50-9.51, 11.7-11.8).

Still another aspect provides an expression vector comprising the polynucleotide. Here, contents regarding "TGF-β" and "peptide" are as described above.

As used herein, the term "expression vector" refers to a recombinant vector that is introduced into a suitable host cell and can express a protein of interest, and to a gene construct comprising essential regulatory elements operably linked to express a gene insert. The term "operably linked" as used herein means that a nucleic acid expression regulatory sequence and a nucleic acid sequence encoding a protein of interest are functionally linked to perform a general function. Operable linkage with a recombinant vector may be performed using genetic recombinant techniques well known in the art, and site-specific DNA cleavage and ligation may be easily performed using enzymes commonly known in the art.

The suitable expression vector of the present invention may comprise a signal sequence for membrane targeting or secretion, in addition to expression control elements such as a promoter, an initiation codon, a termination codon, a polyadenylation signal, and an enhancer. The initiation and termination codons are generally considered to be part of the nucleotide sequence encoding an immunogenic protein of interest, must exhibit activity in a subject when the gene construct is administered, and must be in frame with the coding sequence. Promoters may generally be constitutive or inducible. Examples of promoters for prokaryotic cells include, but are not limited to, lac, tac, T3 and T7 promoters, and examples of promoters for eukaryotic cells include, but are not limited to, a monkey virus 40 (SV40) promoter, a mouse mammary tumor virus (MMTV) promoter, a human immunodeficiency virus (HIV) promoter such as a long terminal repeat (LTR) promoter of HIV, a Moloney virus promoter, a cytomegalovirus (CMV) promoter, an Epstein Barr virus (EBV) promoter, a Rous sarcoma virus (RSV) promoter, a β-actin promoter, and promoters derived from human hemoglobin, human muscle creatine, and human metallothionein.

In addition, the expression vector may comprise a selectable marker for selecting host cells containing the vector. The selectable marker is for selecting cells transformed with the vector. For the selectable marker, markers may be used which confer a selectable phenotype, such as drug resistance, auxotrophy, resistance to cytotoxic agents, or expression of surface proteins. In an environment treated with a selective agent, only cells expressing a selection marker survive, which allows for selection of transformed cells. In addition, when the vector is a replicable expression vector, such a vector may contain a replication origin that is a specific nucleic acid sequence from which replication is initiated.

As a recombinant expression vector for insertion of a foreign gene, various forms of vectors such as plasmids, viruses, and cosmids may be used. The type of recombinant expression vector is not particularly limited as long as the vector functions to express a desired gene and produce a desired protein in various host cells, including prokaryotic cells and eukaryotic cells. Specifically, the recombinant expression vector may be a vector capable of producing a large amount of foreign protein that is in a form similar to its natural state while having a promoter with strong activity and strong expression capacity.

Various combinations of hosts and vectors may be used to express the peptide of the present invention. Expression vectors suitable for eukaryotic hosts include, but are not limited to, expression regulatory sequences derived from SV40, bovine papillomavirus, adenovirus, adeno-associated virus, cytomegalovirus, and retrovirus. Expression vectors that may be used in bacterial hosts include, but are not limited to, bacterial plasmids obtained from *Escherichia coli*, such as pET, pRSET, pBluescript, pGEX2T, pUC vector, colE1, pCR1, pBR322, pMB9, and derivatives thereof; plasmids having a wider host range, such as RP4; phage DNAs that may be exemplified by phage lambda derivatives such as λgt10, λgt11, and NM989; and other DNA phages such as M13 and filamentous single-stranded DNA phages. Expression vectors useful for yeast cells include 2-micron plasmids and derivatives thereof, and expression vectors useful for insect cells include pVL941.

Yet another aspect provides a pharmaceutical composition for treating or preventing TGF-β-related disease, the pharmaceutical composition containing, as an active ingredient, the peptide of the present invention, the polynucleotide of the present invention, or the expression vector of the present invention. Here, contents regarding the "TGF-β" and "peptide" are as described above.

As used herein, the term "TGF-β-related disease" or "TGF-β disease" refers to any disorder, disease or condition that would benefit from treatment with the peptide of the present invention that inhibits the TGF-β signaling pathway. This includes chronic and acute disorders or diseases including those pathological conditions that predispose the subject to the disease in question.

The TGF-β-related diseases include diseases characterized by accumulation of extracellular matrix, diseases caused by circulating TGF-β or TGF-β activated at a local site, diseases caused by suppression of the immune system due to endogenous TGF-β production, acute immune deficiencies resulting from severe injuries, burns, and illnesses such as viral or bacterial infections, multi-organ systemic illnesses due to TGF-beta production or overproduction, and TGF-beta-producing tumors.

Examples of the TGF-β-related diseases include, but are not limited to, cancer, brain nerve diseases (e.g., Alzheimer's dementia and Huntington's disease), fibrotic skin diseases (e.g., scleroderma, CNS pathology scar tissue, skin scarring, keloid scarring, and neural scarring), fibrotic diseases of the lungs, liver and kidneys (e.g., chronic hepatic fibrosis, acute liver injury, interstitial lung and renal fibrosis, and liver cirrhosis), cystic fibrosis, cardiac fibrosis, atherosclerosis and arteriosclerosis, systemic sclerosis, and ocular fibrosis.

As used herein, the term "treatment" refers to any action that alleviates or beneficially changes symptoms of the disease of interest by administration of the composition of the present invention.

As used herein, the term "prevention" refers to any action that suppresses or delays the onset of the disorder or disease of the interest by administering the composition of the present invention.

The pharmaceutical composition may contain a pharmaceutically acceptable carrier. The "pharmaceutically acceptable carrier" refers to a carrier or diluent that does not cause significant irritation to an organism and does not abrogate the biological activity and properties of the administered compound. Here, "pharmaceutically acceptable" means that the carrier does not inhibit the activity of the active ingredient and does not have toxicity that exceeds the toxicity to which the subject can adapt.

A type of carrier that may be used in the present invention may be any carrier that is commonly used in the art and is pharmaceutically acceptable. Non-limiting examples of the carrier include saline, sterile water, Ringer's solution, buffered saline, albumin injection solution, dextrose solution, maltodextrin solution, glycerol, ethanol, and the like. These carriers may be used alone or in combination of two or more. The pharmaceutical composition may contain a pharmaceutically acceptable carrier in addition to the active ingredient and may be prepared in an oral dosage form or a parenteral dosage form depending on the route of administration by a conventional method known in the art.

For use, the pharmaceutical composition may be formulated in the form of oral formulations such as powders, granules, tablets, capsules, suspensions, emulsions, syrups, and aerosols, preparations for external use, suppositories, and sterile injectable solutions, according to the respective conventional methods. The pharmaceutical composition of the present invention may be formulated with commonly used diluents or excipients, such as fillers, extenders, binders, wetting agents, disintegrants, or surfactants.

When the pharmaceutical composition is prepared in an oral dosage form, it may be formulated with suitable carriers in the form of powders, granules, tablets, pills, sugar-coated tablets, capsules, liquids, gels, syrups, suspensions, wafers or the like, according to a method known in the art. Examples of suitable pharmaceutically acceptable carriers include sugars such as lactose, glucose, sucrose, dextrose, sorbitol, mannitol, and xylitol, starches such as corn starch, potato starch, and wheat starch, celluloses such as cellulose, methylcellulose, ethyl cellulose, sodium carboxymethyl cellulose, and hydroxypropyl methyl cellulose, polyvinyl pyrrolidone, water, methylhydroxybenzoate, propylhydroxybenzoate, magnesium stearate, mineral oils, malt, gelatin, talc, polyols, vegetable oils, etc. For formulation, the pharmaceutical composition may be formulated to contain diluents and/or excipients such as fillers, extenders, binders, wetting agents, disintegrants, and surfactants, if necessary.

When the pharmaceutical composition is prepared in parenteral dosage forms, it may be formulated with a suitable carrier in the form of injectable preparations, transdermal preparations, nasal inhalation preparations, and suppositories according to a method known in the art. When the pharmaceutical composition is formulated in the form of injectable preparations, suitable carriers include sterile water, ethanol, polyols such as glycerol or propylene glycol, or mixtures thereof, and preferably, Ringer's solution, PBS (phosphate buffered saline) containing triethanolamine, sterile water for injection, or an isotonic solution such as 5% dextrose may be used. When the pharmaceutical composition is formulated in the form of transdermal preparations, it may be formulated in the form of ointments, creams, lotions, gels, solutions for external use, pastes, liniments, air rolls, or the like. When the pharmaceutical composition is formulated in the form of nasal inhalation preparations, it may be formulated in the form of aerosol sprays using a suitable propellant such as dichlorofluoromethane, trichlorofluoromethane, dichlorotetrafluoroethane, or carbon dioxide. When the pharmaceutical composition is formulated in the form of suppositories, usable bases for the suppositories include witepsol, tween 61, polyethylene glycol, cacao butter, laurin fat, polyoxyethylene sorbitan fatty acid ester, polyoxyethylene stearate, sorbitan fatty acid ester, and the like.

The pharmaceutical composition may be administered in a pharmaceutically effective amount. As used herein, the term "pharmaceutically effective amount" refers to an amount sufficient to treat or prevent a disease at a reasonable benefit/risk ratio applicable to any medical treatment or prevention. The effective dose level may be determined depending on factors, including the severity of the disease, the activity of the drug, the patient's age, body weight, health and sex, patient's sensitivity to the drug, the time of administration of the used composition of the present invention, the route of administration, excretion rate, the duration of treatment, and drugs used in combination or simultaneously with the composition of the present invention, as well as other factors well known in the medical field. The pharmaceutical composition may be administered alone or in combination with known components known to exhibit therapeutic effects against the disease of interest. It is important to administer the pharmaceutical composition in the minimum amount that can exhibit the maximum effect without causing side effects, in view of all the above-described factors.

The dosage of the pharmaceutical composition can be determined by those skilled in the art in consideration of the purpose of use, the severity of the disease, the patient's age, weight, sex, and medical history, the type of substance used as an active ingredient, or the like. For example, the pharmaceutical composition of the present invention may be administered at a dose of about 0.1 ng/kg to about 1,000 mg/kg, preferably 1 ng/kg to about 100 mg/kg, for an adult, and the frequency of administration of the composition of the present invention may be once a day or several times a day at divided doses, without being particularly limited thereto. The dosage or frequency of administration is not intended to limit the scope of the present application in any way.

Still yet another aspect provides a method for treating or preventing TGF-β-related disease, the method comprising a step of administering to a subject the pharmaceutical composition for treating or preventing TGF-β-related disease. Here, contents regarding "TGF-β-related disease" and "pharmaceutical composition" are as described above.

As used herein, the term "subject" includes, without limitation, mammals, including mice, livestock, and humans, birds, reptiles, farmed fish, etc., that have or are at risk of developing TGF-β-related disease.

The pharmaceutical composition may be administered once or multiple times in a pharmaceutically effective amount. In this case, the composition may be formulated and administered in the form of a liquid, powder, aerosol, injection, Ringer's solution, capsule, pill, tablet, suppository or patch. The pharmaceutical composition for preventing or treating cancer may be administered via any general administration route as long as it can reach the target tissue.

The pharmaceutical composition may be administered through routes, including intraperitoneal, intravenous, intramuscular, subcutaneous, intradermal, transdermal patch, oral, intranasal, intrapulmonary, and intrarectal administration routes, depending on the purpose, without being particularly limited thereto. However, the pharmaceutical composition may be administered orally in an unformulated form, and since the active ingredient of the pharmaceutical composition can be denatured or decomposed by gastric acid, the active ingredient of the composition for oral administration may be coated or the composition may be administered orally in a formulated form or an oral patch form so as to be prevented from degradation in the stomach. In addition, the composition of the present invention may also be administered using any device capable of delivering the active ingredient to target cells.

According to one embodiment of the present invention, the peptide contained in the composition is capable of inhibiting TGF-β signaling by binding to TGF-β receptors or by reducing the expression level or extracellular release of TGF-β, thereby effectively treating or preventing TGF-β-related disease.

A further aspect provides a pharmaceutical composition for treating or preventing cancer, the pharmaceutical composition containing, as an active ingredient, the peptide of the present invention, the polynucleotide of the present invention, or the expression vector of the present invention. Here, contents regarding "TGF-β", "peptide" and "pharmaceutical composition" are as described above.

As used herein, the term "cancer" refers to a tumor that grows abnormally due to autonomous overgrowth of body tissue, or a disease that forms the tumor. The cancer may be a cancer expressing TGF-β, more preferably a cancer characterized by excessive activation of TGF-β. Specifically, the cancer may be liver cancer, lung cancer, pancreatic cancer, non-small cell lung cancer, colon cancer, bone cancer, skin cancer, head or neck cancer, skin or intraocular melanoma, uterine cancer, ovarian cancer, rectal cancer, gastric cancer, perianal cancer, colon cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary central nervous system lymphoma, spinal cord tumor, brainstem glioma, or pituitary adenoma, without being limited thereto.

Another further aspect provides a method for treating or preventing cancer, the method comprising a step of administering to a subject the pharmaceutical composition for preventing or treating cancer. Here, contents regarding to the "cancer" and the "pharmaceutical composition" are as described above.

As used herein, the term "subject" includes, without limitation, mammals, including mice, livestock, and humans, birds, reptiles, farmed fish, etc., that have or are at risk of developing cancer disease.

According to one embodiment of the present invention, the peptide contained in the composition is capable of inhibiting TGF-β signaling by binding to TGF-β receptors or by reducing the expression level or extracellular release of TGF-β. Accordingly, the composition is capable of effectively inhibiting the TGF-β-related signaling pathway playing an important role in the tumorigenesis process or cancer microenvironment regulation, and thus can be used to treat or prevent cancer.

### Advantageous Effects

The novel peptide of the present invention is capable of inhibiting the TGF-β signaling pathway, and thus can effectively inhibit TGF-β-related disease and tumorigenesis processes such as TGF-β-induced tumor growth and metastasis. Therefore, the composition containing the peptide has the advantage of being able to effectively treat or prevent TGF-β-related disease, and even cancer.

### Brief Description of Drawings

FIG. 1 summarizes the sequences of candidate peptides for inhibiting TGF-β signaling.
FIG. 2 shows the results of analyzing the binding of the peptides of the present invention to a TGF-β receptor.
FIG. 3 shows time-dependent changes in the expression levels of pSmad2/3 level in Bxpc3 and Aspc1 cell lines after TGF-β treatment.
FIG. 4 shows the expression levels of TGF-β in pancreatic cancer cell lines.
FIG. 5 shows the expression levels of TGF-β receptors in pancreatic cancer cell lines.
FIG. 6 shows changes in the concentrations of TGF-β cytokines after treatment with the peptide of the present invention.
FIG. 7 shows changes in the expression level of pSmad2/3 in the pancreatic cancer cell line Aspc1 after treatment with the peptide of the present invention.
FIG. 8 shows changes in the expression level of pSmad2/3 in the pancreatic cancer cell line Bxpc3 after treatment with the peptide of the present invention.
FIG. 9 shows changes in the expression level of pSmad2/3 in the pancreatic cancer cell line Panc1 after treatment with the peptide of the present invention.
FIG. 10 shows changes in cancer cell proliferation ability in the pancreatic cancer cell line Aspc1 after treatment with the peptide of the present invention.

### Best Mode

One aspect provides a novel peptide described below, a polynucleotide encoding the same, or an expression vector comprising the polynucleotide.

In one embodiment of the present invention, the peptide may comprise an amino acid sequence represented by the following Formula 1, which is set forth in SEQ ID NO: 16.

[Formula 1] F-X₁-L-G-P-X₂-P-Y-I-W-X₃-L-D-T

wherein X₁, X₂ and X₃ may be each independently cysteine (C) or serine (S), and specifically, X₁-X₂-X₃ may be C-C-S, C-C-C, or S-S-S.

The peptide may additionally comprise any amino acid or peptide. Specifically, the peptide may comprise a peptide in which any additional amino acid or peptide is linked directly to one terminus, preferably the C-terminus, of the amino acid sequence represented by Formula 1. The additional amino acid or peptide may be alanine (A), phenylalanine (F), valine (V), leucine (L), serine (S), or a combination thereof, and non-limiting examples thereof include alanine (A), phenylalanine (F), valine-leucine-serine-leucine (V-L-S-L), or valine-leucine-serine-phenylalanine (V-L-S-F).

The peptide may comprise the amino acid sequence of at least one of SEQ ID NOs: 1 to 8, and specifically, may consist of the amino acid sequence of one of SEQ ID NOs: 1 to 8.

In one embodiment of the present invention, the peptide may comprise an amino acid sequence represented by the following Formula 2, which is represented by SEQ ID NO: 17.

[Formula 2] A-H-C-S-C-D

The peptide may additionally comprise any amino acid or peptide. Specifically, the peptide may comprise a peptide in which any additional amino acid or peptide is linked directly to one terminus, preferably the C-terminus, of the amino acid sequence represented by Formula 2. The additional amino acid or peptide may be serine (S), arginine (R), aspartic acid (D), asparagine (N), glycine (G), alanine (A), phenylalanine (F), or a combination thereof, and non-limiting examples thereof include serine-arginine-aspartic acid-asparagine (S-R-D-N), or glycine-alanine-phenylalanine-alanine (G-A-F-A).

The peptide may comprise the amino acid sequence of at least one of SEQ ID NOs: 9 and 10, and specifically, may consist of the amino acid sequence of one of SEQ ID NOs: 9 and 10.

In one embodiment of the present invention, the peptide may comprise an amino acid sequence represented by the following Formula 3, which is represented by SEQ ID NO: 18.

[Formula 3] T-I-V-Y-Y-V-X₄-X₅-K-P-K-V-E-Q

wherein X₄ may be glycine (G), histidine (H) or valine (V), X₅ may be arginine (R), leucine (L), or isoleucine (I), and specifically, X₄-X₅ may be G-R, G-L, H-I, or V-I.

The peptide may comprise the amino acid sequence of at least one of SEQ ID NOs: 11 to 14, and specifically, may consist of the amino acid sequence of one of SEQ ID NOs: 11 to 14.

Another aspect provides the use of the peptide, the polynucleotide or the expression vector for preventing or treating a TGF-β-related disease, in particular, a cancer expressing TGF-β.

Examples of the TGF-β-related disease include, but are not limited to, cancer, brain nerve diseases (e.g., Alzheimer's dementia and Huntington's disease), fibrotic skin diseases (e.g., scleroderma, CNS pathology scar tissue, skin scarring, keloid scarring, and neural scarring), fibrotic diseases of the lungs, liver and kidneys (e.g., chronic hepatic fibrosis, acute liver injury, interstitial lung and renal fibrosis, and liver cirrhosis), cystic fibrosis, cardiac fibrosis, atherosclerosis and arteriosclerosis, systemic sclerosis, and ocular fibrosis.

The cancer may be liver cancer, lung cancer, pancreatic cancer, non-small cell lung cancer, colon cancer, bone cancer, skin cancer, head or neck cancer, skin or intraocular melanoma, uterine cancer, ovarian cancer, rectal cancer, gastric cancer, perianal cancer, colon cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary central nervous system lymphoma, spinal cord tumor, brainstem glioma, or pituitary adenoma, without being limited thereto.

### Mode for Invention

Hereinafter, the present invention will be described in more detail with reference to examples. However, these examples are for illustrative purposes only and the scope of the present invention is not limited to these examples.

### Example 1: Design of peptides for inhibiting TGF-β-signaling

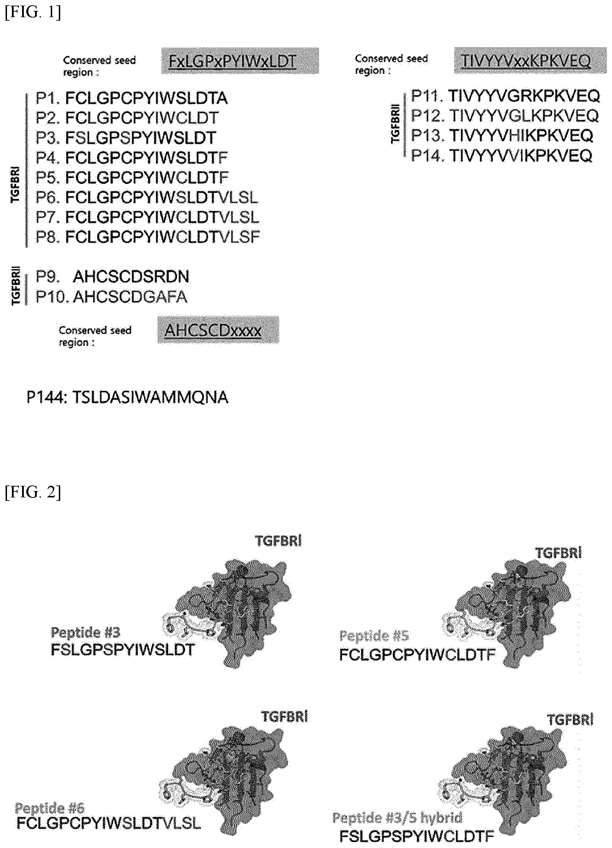

To develop the TGF-β signaling inhibitory peptides of the present invention, 14 candidate amino acid sequences derived from TGF-β that target the TGF-β receptors were designed and produced. Specific information on the candidate sequences is shown in Table 1 below and FIG. 1.

**[Table 1]**

| **Candidate peptide** | **Amino acid sequence** | **Target** | **SEQ ID NO.** |
|---|---|---|---|
| P1 | FCLGPCPYIWSLDTA | TGFβRI/II | 1 |
| P2 | FCLGPCPYIWCLDT | TGFβRI/II | 2 |
| P3 | FSLGPSPYIWSLDT | TGFβRI/II | 3 |
| P4 | FCLGPCPYIWSLDTF | TGFβRI/II | 4 |
| P5 | FCLGPCPYIWCLDTF | TGFβRI/II | 5 |
| P6 | FCLGPCPYIWSLDTVLSL | TGFβRI/II | 6 |
| P7 | FCLGPCPYIWCLDTVLSL | TGFβRI/II | 7 |
| P8 | FCLGPCPYIWCLDTVLSF | TGFβRI/II | 8 |
| P9 | AHCSCDSRDN | TGFβRI/II | 9 |
| P10 | AHCSCDGAFA | TGFβRI/II | 10 |
| P11 | TIVYYVGRKPKVEQ | TGFβRII | 11 |
| P12 | TIVYYVGLKPKVEQ | TGFβRII | 12 |
| P13 | TIVYYVHIKPKVEQ | TGFβRII | 13 |
| P14 | TIVYYVVIKPKVEQ | TGFβRII | 14 |
| P144 (control) | TSLDASIWAMMQNA | TGFβRII | 15 |

For the production of the candidate peptides, a domain capable of binding to TGF-β receptor 1 (TGFBR1) was predicted using the protein crystal structure (PDB: 3KFD) registered in the protein data bank (http://www.rcsb.org). Based on this prediction, specific peptides were designed, and the possibility of binding thereof was examined through the molecular modeling program Autodock, and then the predicted structures were produced. As a result, it was confirmed that, typically, P3, P5, P3/5 hybrid and P6 peptides could bind to TGF-β receptor 1 (FIG. 2).

Based on the above results, it can be seen that the candidate peptides produced in the present invention can inhibit the signaling pathway induced by TGF-β by binding to the TGF-β receptor.

### Example 2: Establishment of conditions for evaluating TGF-β-signaling inhibitory effect

In order to establish conditions for evaluating the TGF-β signaling inhibitory effects of the candidate peptides produced in Example 1, the following experiment was conducted.

It is known that the TGF-β signaling pathway proceeds from TGF-β receptor, and when the TGF-β receptor is activated by TGF-β, the TGF-β signaling pathway proceeds through a process in which Smad2/3 protein is phosphorylated. Accordingly, activation or inhibition of the TGF-β signaling pathway can be confirmed through changes in the expression level of phospho-Smad2/3 (p-Smad2/3), and thus conditions for inducing p-Smad2/3 by TGF-β treatment were examined.

Specifically, in order to analyze the time-dependent expression of phospho-Smad2/3 in cells by Western blotting assay, each of pancreatic cancer cell lines Bxpc3 and Aspc1 was treated with TGF-β cytokine at 50 ng/ml, and then cells obtained at different times were lysed by adding a cell lysis buffer containing a protease inhibitor (Thermo Scientific), and then sampled, thus preparing samples for Western blotting. Each of the samples was electrophoresed on SDS-PAGE gel, transferred to nitrocellulose membranes, and then blocked using skim milk powder. Next, the membranes were incubated with a primary antibody against Smad2/3 or pSmad2/3 at 4°C for one day, washed, and then incubated with a horseradish peroxidase-conjugated secondary antibody capable of binding to the primary antibody at room temperature for 2 hours. Then, the membranes were visualized by chemiluminescence using a Western ECL substrate (Thermo Scientific), and the obtained luminescence images were analyzed with Chemidoc (BioRad).

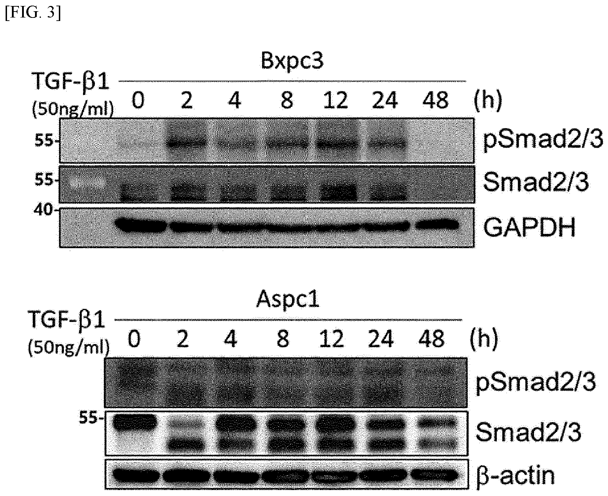

As a result, it was confirmed that the expression levels of pSmad2/3 in the Bxpc3 and Aspc1 cell lines were high at 12 to 24 hours after treatment with TGF-β (FIG. 3). Thus, in the following experiment, whether the TGF-β signaling pathway was inhibited was examined by analyzing the expression level of pSmad2/3 at 24 hours after treatment with TGF-β.

Next, in order to analyze the expression levels of TGF-β in pancreatic cancer cell lines, each of the pancreatic cancer cell lines Bxpc3, Aspc1, Panc1, Capan2 and miapaca2 and the cancer cell line SNU213 derived from a patient was cultured in a 6-well plate at a cell density of 1 × 10⁶ cells/well for 48 hours, and then the concentrations of the TGF-β cytokine in the culture media were analyzed by ELISA technique.

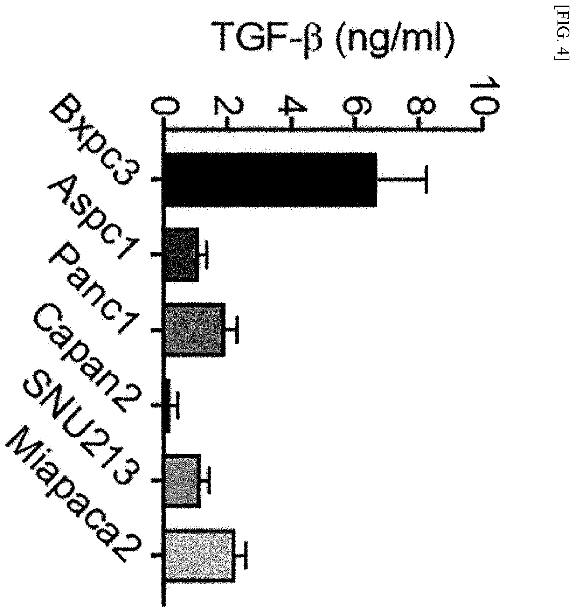

As a result, it was confirmed that TGF-β cytokine levels were expressed in the BxPC3, AsPC1, Panc1, Capan2, Miapaca2 and SNU213 cells (FIG. 4). Thereby, it can be seen that the cell lines can be used in an experiment for evaluating the effect of inhibiting TGF-β signaling through a mechanism of inhibiting TGF-β expression level.

Next, in order to confirm the expression of TGF-β receptors (TGFBR) to which TGF-β in pancreatic cancer cell lines bind, expression of TGFBR1, TGFBR2 and TGFBR3 receptors in the pancreatic cancer cell lines BxPC3, AsPC1, and Miapaca2 was analyzed by Western blotting assay. Specifically, after each cell line was lysed by adding a cell lysis buffer and then sampled, thus preparing samples for Western blotting. Each sample was analyzed by the Western blotting method described in Example 2, and expression of the receptors was analyzed using primary antibodies capable of detecting TGFBR1, TGFBR2 and TGFBR3, respectively.

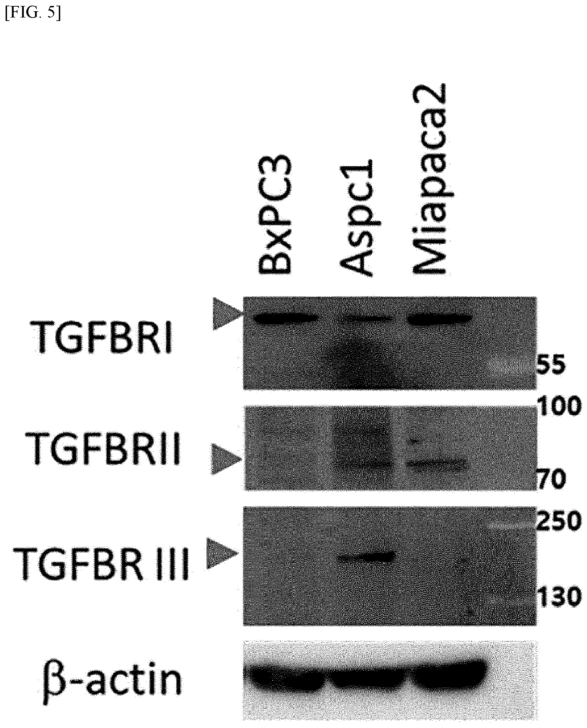

As a result, it was confirmed that the pancreatic cancer cell line AsPC1 generally exhibited high expression levels of TGFBR1, TGFBR2 and TGFBR3, and the other cell lines also expressed TGFBR (FIG. 5). Thereby, it can be seen that the cell lines can be used in an experiment for evaluating the effect of inhibiting TGF-β signaling through a mechanism of inhibiting the binding between TGF-β and the TGF-β receptors.

### Example 3: Evaluation of effects of candidate peptides on inhibition of TGF-β expression level

In order to examine whether the candidate peptides produced in Example 1 can inhibit the expression level of extracellular TGF-β, the following experiment was conducted.

Specifically, the pancreatic cancer cell line Aspc1 was seeded in a 96-well plate at a density of 2 × 10⁴ cells/well, cultured for one day, and then treated with 50 ng/ml of TGF-β or TGF-β plus inhibitor (candidate peptide and control peptide) for 48 hours, and then the concentrations of the TGF-β cytokine in the cancer cell culture media were analyzed by ELISA technique using a TGF-β detection ELISA kit. The concentration of the extracellularly released TGF-β cytokine was determined by measuring the absorbance with a multiplate reader, and the results were graphed.

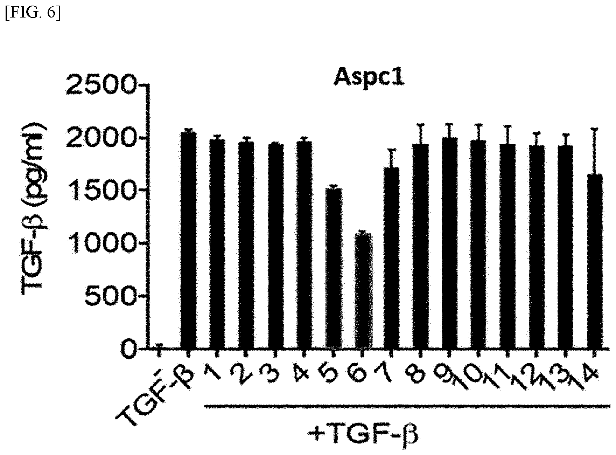

As a result, it was confirmed that P5, P6, P7 and P14 peptides among the candidate peptides directly inhibited the extracellular release of TGF-β cytokine itself from the Aspc1 cells (FIG. 6). Based on the above results, it can be seen that the TGF-β inhibitory peptides of the present invention can inhibit the TGF-β signaling pathway by inhibiting the expression level of TGF-β, specifically, the extracellular release of TGF-β itself.

### Example 4: Evaluation of effects of candidate peptides on inhibition of TGF-β signaling

In order to evaluate the TGF-β signaling inhibitory effects of the candidate peptides produced in Example 1, the following experiment was conducted.

Specifically, each of the pancreatic cancer cell lines Aspc1, Bxpc3 and Panc1 was seeded in a 6-well plate at a density of 1 × 10⁶ cells/well and cultured for one day. The next day, the cells were treated with TGF-β or TGF-β plus candidate peptide for 24 hours (control: 15% ACN - candidate peptide solvent, DMSO - P144 peptide solvent, P144), and the expression levels of pSmad2/3 and total Smad2/3 were analyzed by the Western blotting method described in Example 2.

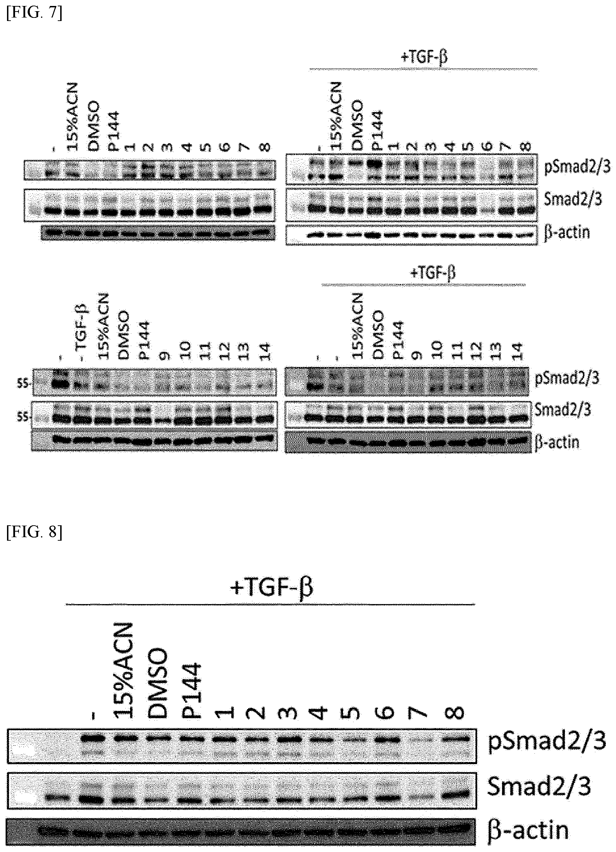

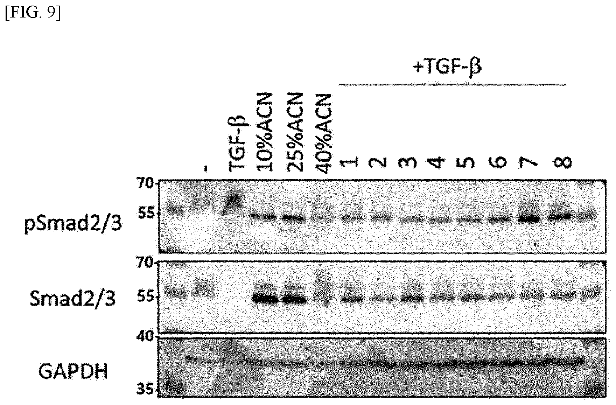

As a result, it was confirmed that, in the case of the Aspc1 cells, the peptides according to the present invention inhibited the expression level of pSmad2/3, and among these peptides, the P6 peptide had the best ability to inhibit the expression level of pSmad2/3 (FIG. 7). In addition, it was confirmed that, in the case of the Bxpc3 and Panc1 cell lines, the peptides according to the present invention significantly inhibited the expression level of pSmad2/3 (FIGS. 8 and 9). Based on the above results, it can be seen that the TGF-β signaling inhibitory peptides produced in the present invention can actually effectively inhibit TGF-β signaling.

### Example 5: Confirmation of inhibition of cancer cell growth by candidate peptides

In order to confirm the effect of inhibiting cancer cell proliferation through inhibition of TGF-β signaling by the candidate peptides produced in Example 1, the following experiment was conducted.

Specifically, the pancreatic cancer cell line Aspc1 was seeded in a 96-well plate at a density of 2 × 10⁴ cells/well, cultured for one day, and then treated with 50 ng/ml of TGF-β or TGF-β plus inhibitor (candidate peptide, galunicertip or LY2109761) for 24 hours. Next, CCK-8 assay reagent (Sigma) was added to each well, and the percentage of viable cells was determined by measuring the absorbance with a multiplate reader, and the results were graphed. Gaunisertib and LY2109761, known TGF-β inhibitors, were used at a concentration of 100 µM.

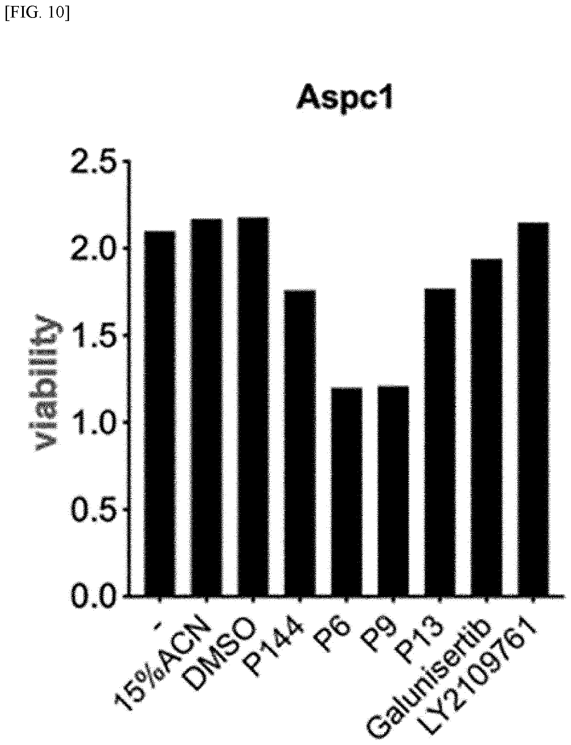

As a result, it could be confirmed that when the cancer cell line was treated with the peptides according to the present invention, previously confirmed to inhibit TGF-β signaling, the proliferation of the cancer cells was inhibited (FIG. 10).

Based on the above results, it can be seen that the TGF-β signaling inhibitory peptides produced in the present invention can inhibit the proliferation of cancer cells through a mechanism of inhibiting TGF-β signaling.

The above description of the present invention is for illustrative purposes, and those skilled in the art to which the present invention pertains will appreciate that the present invention may be easily modified into other specific forms without changing the technical spirit or essential features of the present invention. Therefore, the embodiments described above are considered to be illustrative in all respects and not restrictive

### Industrial Applicability

The present invention relates to a peptide capable of inhibiting TGF-β signaling and a method of treating or preventing TGF-β signaling-related disease, and even, TGF-β-expressing cancer, by using the peptide.

## Claims

1. A peptide derived from transforming growth factor-β (TGF-β) or an analog thereof.

2. The peptide of claim 1, comprising an amino acid sequence of the following Formula 1, which is set forth in SEQ ID NO: 16, or an amino acid sequence having a homology of at least 80% to the sequence:
[Formula 1] F-X₁-L-G-P-X₂-P-Y-I-W-X₃-L-D-T
wherein X₁, X₂ and X₃ are each independently cysteine (C) or serine (S).

3. The peptide of claim 2, further comprising alanine (A), phenylalanine (F), valine (V), leucine (L), serine (S), or a combination thereof at one terminus of the amino acid sequence of Formula 1.

4. The peptide of claim 3, further comprising alanine (A), phenylalanine (F), valine-leucine-serine-leucine (V-L-S-L) or valine-leucine-serine-phenylalanine (V-L-S-F) at the C-terminus of the amino acid sequence of Formula 1.

5. The peptide of claim 2, comprising the amino acid sequence of at least one of SEQ ID NOs: 1 to 8 or an amino acid sequence having a homology of at least 80% to the sequence.

6. The peptide of claim 1, comprising an amino acid sequence of the following Formula 2, which is set forth in SEQ ID NO: 17, or an amino acid sequence having a homology of at least 80% to the sequence:
[Formula 2] A-H-C-S-C-D

7. The peptide of claim 6, further comprising serine (S), arginine (R), aspartic acid (D), asparagine (N), glycine (G), alanine (A), phenylalanine (F), or a combination thereof at one terminus of the amino acid sequence of Formula 2.

8. The peptide of claim 7, further comprising serine-arginine-aspartic acid-asparagine (S-R-D-N) or glycine-alanine-phenylalanine-alanine (G-A-F-A) at the C-terminus of the amino acid sequence of Formula 2.

9. The peptide of claim 6, comprising the amino acid sequence of at least one of SEQ ID NOs: 9 and 10 or an amino acid sequence having a homology of at least 80% to the sequence.

10. The peptide of claim 1, comprising an amino acid sequence of the following Formula 3, which is set forth in SEQ ID NO: 18, or an amino acid sequence having a homology of at least 80% to the sequence:
[Formula 3] T-I-V-Y-Y-V-X₄-X₅-K-P-K-V-E-Q
wherein X₄ is glycine (G), histidine (H) or valine (V), and X₅ is arginine (R), leucine (L) or isoleucine (I).

11. The peptide of claim 10, comprising the amino acid sequence of at least one of SEQ ID NOs: 11 to 14 or an amino acid sequence of at least 80% to the sequence.

12. The peptide of claim 10, which inhibits TGF-β signaling.

13. A polynucleotide encoding the peptide of any one of claims 1 to 12.

14. An expression vector comprising the polynucleotide of claim 13.

15. A pharmaceutical composition for preventing or treating TGF-β-related disease, the pharmaceutical composition containing, as an active ingredient, the peptide of any one of claims 1 to 12, a polynucleotide encoding the same, or an expression vector comprising the polynucleotide.

16. The pharmaceutical composition of claim 15, wherein TGF-β-related disease is cancer, brain nerve disease, fibrotic skin disease, pulmonary fibrosis, hepatic fibrosis, renal fibrosis, cystic fibrosis, cardiac fibrosis, atherosclerosis, arteriosclerosis, systemic sclerosis, or ocular fibrosis.

17. A method for preventing or treating TGF-β-related disease, the method comprising a step of administering to a subject a pharmaceutical composition containing, as an active ingredient, the peptide of any one of claims 1 to 12, a polynucleotide encoding the same, or an expression vector comprising the polynucleotide.

18. The method of claim 17, wherein TGF-β-related disease is cancer, brain nerve disease, fibrotic skin disease, pulmonary fibrosis, hepatic fibrosis, renal fibrosis, cystic fibrosis, cardiac fibrosis, atherosclerosis, arteriosclerosis, systemic sclerosis, or ocular fibrosis.


Sequence Listing Free Text

<110>	Korea Institute of Science and Technology

<120>	Novel peptides capable of inhibiting TGF-beta signaling and uses
thereof

<130>	OP21-013PCT

<150>	KR 10-2020-0182441
<151>	2020-12-23

<160>	18

<170>	KoPatentIn 3.0

<210>	1
<211>	15
<212>	PRT
<213>	Artificial Sequence

<220>
<223>	P1

<400>	1
Phe Cys Leu Gly Pro Cys Pro Tyr Ile Trp Ser Leu Asp Thr Ala
1               5                   10                  15

<210>	2
<211>	14
<212>	PRT
<213>	Artificial Sequence

<220>
<223>	P2

<400>	2

Phe Cys Leu Gly Pro Cys Pro Tyr Ile Trp Cys Leu Asp Thr

1               5                   10

<210>	3

<211>	14

<212>	PRT

<213>	Artificial Sequence

<220>

<223>	P3

<400>	3

Phe Ser Leu Gly Pro Ser Pro Tyr Ile Trp Ser Leu Asp Thr

1               5                   10

<210>	4

<211>	15

<212>	PRT

<213>	Artificial Sequence

<220>

<223>	P4

<400>	4
Phe Cys Leu Gly Pro Cys Pro Tyr Ile Trp Ser Leu Asp Thr Phe
1               5                   10                  15

<210>	5
<211>	15
<212>	PRT
<213>	Artificial Sequence

<220>
<223>	P5

<400>	5
Phe Cys Leu Gly Pro Cys Pro Tyr Ile Trp Cys Leu Asp Thr Phe
1               5                   10                  15

<210>	6
<211>	18
<212>	PRT

<213>	Artificial Sequence

<220>

<223>	P6

<400>	6

Phe Cys Leu Gly Pro Cys Pro Tyr Ile Trp Ser Leu Asp Thr Val Leu
1               5                   10                  15

Ser Leu

<210>	7

<211>	18

<212>	PRT

<213>	Artificial Sequence

<220>

<223>	P7

<400>	7

Phe Cys Leu Gly Pro Cys Pro Tyr Ile Trp Cys Leu Asp Thr Val Leu

1                    5                        10                          15

Ser Leu

<210>        8

<211>        18

<212>        PRT

<213>        Artificial Sequence

<220>

<223>        P8

<400>        8

Phe Cys Leu Gly Pro Cys Pro Tyr Ile Trp Cys Leu Asp Thr Val Leu
  1                  5                          10                          15

Ser Phe

<210>        9

<211>        10

<212>	PRT
<213>	Artificial Sequence

<220>
<223>	P9

<400>	9
Ala His Cys Ser Cys Asp Ser Arg Asp Asn
1               5                   10

<210>	10
<211>	10
<212>	PRT
<213>	Artificial Sequence

<220>
<223>	P10

<400>	10
Ala His Cys Ser Cys Asp Gly Ala Phe Ala
1               5                   10

<210>	11
<211>	14
<212>	PRT
<213>	Artificial Sequence

<220>
<223>	P11

<400>	11
Thr Ile Val Tyr Tyr Val Gly Arg Lys Pro Lys Val Glu Gln
1               5                   10

<210>	12
<211>	14
<212>	PRT
<213>	Artificial Sequence

<220>
<223>	P12

<400>	12

Thr Ile Val Tyr Tyr Val Gly Leu Lys Pro Lys Val Glu Gln
1               5                   10

<210>   13
<211>   14
<212>   PRT
<213>   Artificial Sequence

<220>
<223>   P13

<400>   13
Thr Ile Val Tyr Tyr Val His Ile Lys Pro Lys Val Glu Gln
1               5                   10

<210>   14
<211>   14
<212>   PRT
<213>   Artificial Sequence

<220>
<223>   P14

<400>	14

Thr Ile Val Tyr Tyr Val Val Ile Lys Pro Lys Val Glu Gln

1               5                   10

<210>	15

<211>	14

<212>	PRT

<213>	Artificial Sequence

<220>

<223>	P144

<400>	15

Thr Ser Leu Asp Ala Ser Ile Trp Ala Met Met Gln Asn Ala

1               5                   10

<210>	16

<211>	14

<212>	PRT

<213>	Artificial Sequence

<220>

<223>	peptide 1

<220>

<221>	MISC_FEATURE

<222>	(2)

<223>	Xaa is cysteine or serine

<220>

<221>	MISC_FEATURE

<222>	(6)

<223>	Xaa is cysteine or serine

<220>

<221>	MISC_FEATURE

<222>	(11)

<223>	Xaa is cysteine or serine

<400>	16

Phe Xaa Leu Gly Pro Xaa Pro Tyr Ile Trp Xaa Leu Asp Thr

1                    5                         10

<210>    17

<211>    6

<212>    PRT

<213>    Artificial Sequence

<220>

<223>    peptide 2

<400>    17

Ala His Cys Ser Cys Asp

1                    5

<210>    18

<211>    14

<212>    PRT

<213>    Artificial Sequence

<220>

<223>    peptide 3

<220>

<221>	MISC_FEATURE

<222>	(7)

<223>	Xaa is glycine, histidine or valine

<220>

<221>	MISC_FEATURE

<222>	(8)

<223>	Xaa is arginine, leucine or isoleucine

<400>	18

Thr Ile Val Tyr Tyr Val Xaa Xaa Lys Pro Lys Val Glu Gln

1               5                   10